# FASCICULE DE BREVET EUROPEEN

(11) **EP 0 634 911 B1**
(45) Date de publication et mention de la délivrance du brevet: **01.10.1997**
(21) Numéro de dépôt: 93909019.7
(22) Date de dépôt: 09.04.1993
(51) Int. Cl.: A61B 17/58, A61B 17/60

(54) **DISPOSITIF D'OSTEOSYNTHESE RACHIDIENNE**
VORRICHTUNG ZUR OSTEOSYNTHESE VON RÜCKENWIRBELN
SPINAL OSTEOSYNTHESIS DEVICE

(30) Priorité: 10.04.1992 FR 9204449
(43) Date de publication de la demande: 25.01.1995
(73) Titulaire: EUROSURGICAL, F-62217 Beaurains (FR)
(72) Inventeur: MAZEL, Christian, F-92410 Ville-d'Avray (FR)
(74) Mandataire: Martin, Jean-Paul
(86) Numéro de dépôt international: FR9300366
(87) Numéro de publication internationale: WO9320771

(56) Documents cités:
- EP-A- 0 140 790
- EP-A- 0 301 489
- EP-A- 0 443 894
- WO-A-91/16018
- FR-A- 2 309 200
- FR-A- 2 651 992
- US-A- 5 030 220
- US-A- 5 057 109

## Description

La présente invention a pour objet un dispositif d'ostéosynthèse rachidienne destiné au traitement des scolioses, des tumeurs, des fractures et des pathologies dégénératives.

L'ostéosynthèse rachidienne est actuellement réalisée principalement par des dispositifs utilisant des plaques ou des tiges rigides.

On peut citer comme exemples :
les plaques de Steffee, la technique de WILTSE, le mille pattes de Roy Camille, la technique CD, la technique du Texas Scottish Right Hospital.

Ces dispositifs posent généralement des problèmes dans la mise en place des plaques ou des tiges sur les implants d'ancrage osseux. En effet, le positionnement de ces ancrages n'étant pas rectiligne, ni de même orientation dans le plan sagittal, notamment en ce qui concerne les vis vertébrales, il est nécessaire de placer les plaques ou les tiges en force, ce qui est préjudiciable à la bonne tenue des ancrages, ou alors de conformer les tiges, par exemple pour pouvoir réaliser la fixation des éléments d'ancrage.

Avec ces dispositifs, la réduction est souvent difficile par le fait que l'on doit réduire en même temps que l'on fixe les ancrages osseux aux plaques ou aux tiges. Cette réduction se fait difficilement de manière souple.

De plus la courbure des tiges ou des plaques doit généralement être réalisée avant la mise en place sur les éléments d'ancrages osseux, ce qui limite l'optimisation de la réduction.

De l'avis de nombreux chirurgiens, la rigidité trop importante des instrumentations agit de façon néfaste sur la rapidité de la consolidation osseuse de la greffe associée à l'ostéosynthèse rachidienne.

On connaît par ailleurs, par le US-A-5 057 109, des éléments d'ancrage osseux vertébral conformes au préambule de la revendication 1.

L'invention a pour but de proposer un dispositif d'ostéosynthèse à fixation segmentaire utilisable en voie postérieure en bilatéral et en voie antérieure, agencé de manière à :
- faciliter la mise en place des implants grâce à la flexibilité des liaisons longitudinales s'adaptant à tout positionnement des éléments d'ancrage;
- permettre d'affiner la réduction par simple mouvement d'écartement ou de rapprochement et de rigidifier le montage progressivement segment par segment;
   - préserver une élasticité suffisante de manière à favoriser la fusion osseuse de la greffe associée à l'ostéosynthèse;
   - obtenir une instrumentation réduite en encombrement.

Le dispositif d'ostéosynthèse rachidienne selon l'invention est conforme à la partie caractérisante de la revendication 1.

Les tiges flexibles sont de section circulaire ou non, par exemple oblongue et fabriquées en matériau biocompatible à haute limite élastique et haute résistance à la rupture. De plus, ces tiges flexibles peuvent être des tiges pleines ou bien des assemblages de fils comme par exemple des torons.

On comprend qu'un tel dispositif d'étaiement du rachis préserve une certaine élasticité dans la zone arthrodésée, grâce à la mise en oeuvre de tiges métalliques flexibles à haute limite élastique, qui traversent un nombre approprié de connecteurs eux-mêmes solidement ancrés aux vertèbres par des éléments tels que vis et crochets.

Les tiges métalliques ont une section faible par rapport aux tiges des instrumentations postérieures utilisées jusqu'à présent, et une inertie appropriée pour donner l'élasticité à l'ostéosynthèse favorable à la bonne greffe osseuse.

Suivant d'autres caractéristiques de l'invention:
- chaque connecteur comprend un socle dans lequel sont ménagés deux alésages traversés par des tiges flexibles correspondantes, ainsi qu'un trou de réception du corps expansible d'un élément d'ancrage, et ledit connecteur comprend également des moyens de fixation des tiges au socle.
- lesdits moyens de fixation comprennent une bride dans une face de laquelle sont agencées des gouttières complémentaires de gouttières correspondantes ménagées dans une face du socle dans le prolongement des alésages, de telle sorte que les tiges flexibles soient logées dans les gouttières conjuguées de la bride et du socle, la bride étant pourvue d'un organe de serrage sur le socle et de blocage des tiges dans leurs alésages et gouttières, tel qu'une vis traversant le trou lisse de la bride et le trou taraudé du socle.

L'invention sera maintenant décrite en référence aux dessins annexés qui en illustrent plusieurs formes de réalisation à titre d'exemples non limitatifs.

La figure 1 est une vue en élévation d'une première forme de réalisation d'un dispositif d'ostéosynthèse rachidienne selon l'invention, correspondant à un montage lombo-sacré.

La figure 2 est une vue en perspective à échelle agrandie, d'un crochet pédiculaire d'ancrage osseux pouvant faire partie du dispositif de la Fig.1.

La figure 3 est une vue en perspective à échelle agrandie similaire à la Fig.2, d'un crochet laminaire et de sa vis d'expansion.

La figure 4 est une vue en perspective éclatée à échelle agrandie d'un élément d'ancrage osseux selon un troisième mode de réalisation, constitué par une vis pédiculaire et sa vis d'expansion.

La figure 5 est une vue mi-élévation mi-coupe axiale de la tête de la vis de la Fig.4 dans laquelle a été enfoncée la vis d'expansion.

La figure 6 est une vue en perspective éclatée des trois parties constitutives d'un connecteur d'ancrage osseux faisant partie du dispositif de la Fig.1.

La figure 7 est une vue en perspective d'une variante de réalisation du connecteur de la Fig.6.

La figure 8 est une vue en perspective du connecteur de la Fig.6 assemblé à deux tiges flexibles.

La figure 9 est une vue en coupe partielle et élévation longitudinale du connecteur de la Fig.8 équipé d'une vis d'ancrage osseux.

La figure 10 est une vue de dessus en plan du connecteur des Fig.8 et 9.

La figure 11 est une vue en perspective à échelle agrandie d'un connecteur de fixation sacrée faisant partie du dispositif de la Fig.1, et de la tige bouclée sur ce connecteur.

La figure 12 est une vue en coupe longitudinale du connecteur de la Fig.11 suivant 12-12 de la Fig.14, dans un plan contenant les axes parallèles, des deux alésages de ce connecteur.

La figure 13 est une vue de dessus en plan du connecteur de la Fig.11.

La figure 14 est une vue en bout suivant la flèche K de la Fig.13.

La figure 15 est une vue en perspective éclatée d'une autre forme de réalisation de l'élément d'ancrage osseux.

Les figures 16 et 17 sont des vues en perspective à échelle agrandie de deux modes de réalisation d'une plaque d'appui antérieur.

La figure 18 est une vue en élévation d'une instrumentation antérieure comportant des plaques d'appui selon le mode de réalisation de la Fig.16.

La figure 19 est une vue en élévation postérieure de l'instrumentation antérieure de la Fig.18.

La figure 20 est une vue en élévation longitudinale d'un connecteur double muni de vis d'ancrage osseux, ce connecteur double pouvant former un pont au-dessus d'une vertèbre fracturée.

La figure 21 est une vue en plan des deux plaques constitutives du connecteur de la Fig.19.

La figure 22 est une vue en élévation latérale d'une instrumentation postérieure pour vertèbres fracturées, mise en place sur la partie correspondante du rachis et comportant un connecteur double selon les Fig.20 et 21.

La figure 23 est une vue en élévation sagittale d'une instrumentation lombaire constituée d'éléments d'ancrage osseux du type crochets pédiculaires et laminaires conformes aux Fig.2 et 3.

La figure 24 est une vue en coupe partielle du connecteur des Fig.6 à 10 au niveau du trou de passage de la vis d'ancrage osseux.

La figure 25 est une vue en perspective partielle à échelle agrandie, d'une autre forme de réalisation des tiges longitudinales flexibles du dispositif selon l'invention.

La figure 26 est une vue en bout de la tige de la Fig.25.

La figure 27 est une vue analogue à la Fig.25 d'une variante de réalisation de la tige flexible.

La figure 28 est une vue en bout de la tige de la Fig.27.

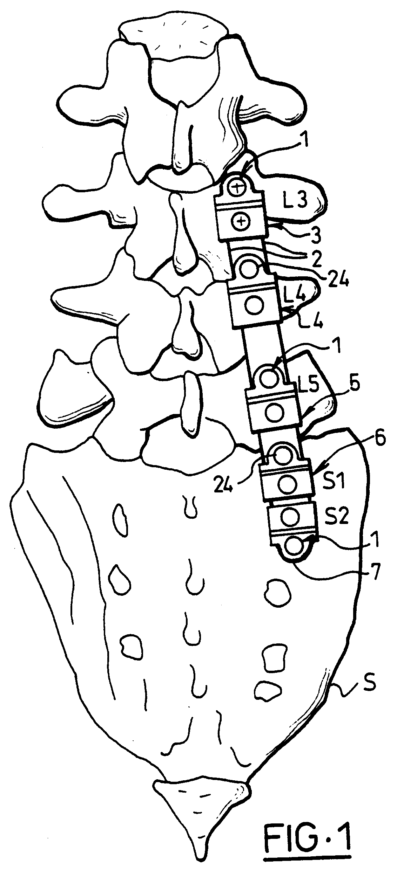

On voit à la Fig.1 un dispositif d'ostéosynthèse rachidienne constitué par une instrumentation lombo-sacrée, s'étendant à partir du sacrum S, en S1, S2, et s'étendant sur les trois premières vertèbres lombaires L5, L4, L3. Ce dispositif comprend des éléments 1 d'ancrage osseux dans le sacrum et dans les vertèbres lombaires, deux tiges longitudinales flexibles 2 à haute limite élastique, de section faible, à peu près parallèles entre elles et qui traversent les connecteurs (3, 4, 5, 6) auxquels elles sont fixées, et qui sont eux-mêmes mécaniquement solidarisés avec les éléments d'ancrage 1.

Les deux tiges 2 sont, dans cette réalisation, constituées par deux parties parallèles d'une seule tige recourbée en épingle, qui traversent les quatre premiers connecteurs 3, 4, 5, 6, le dernier étant ancré dans le sacrum en S1. Les deux tiges se rejoignent en formant une boucle sur un arrondi du connecteur sacré 7, placé en S2.

Les tiges 2 peuvent être en acier à haute limite élastique, par exemple 650 N/mm², et à haute résistance à la rupture (par exemple 1400 N/mm²) leur diamètre pouvant être de 2 ou 3mm environ.

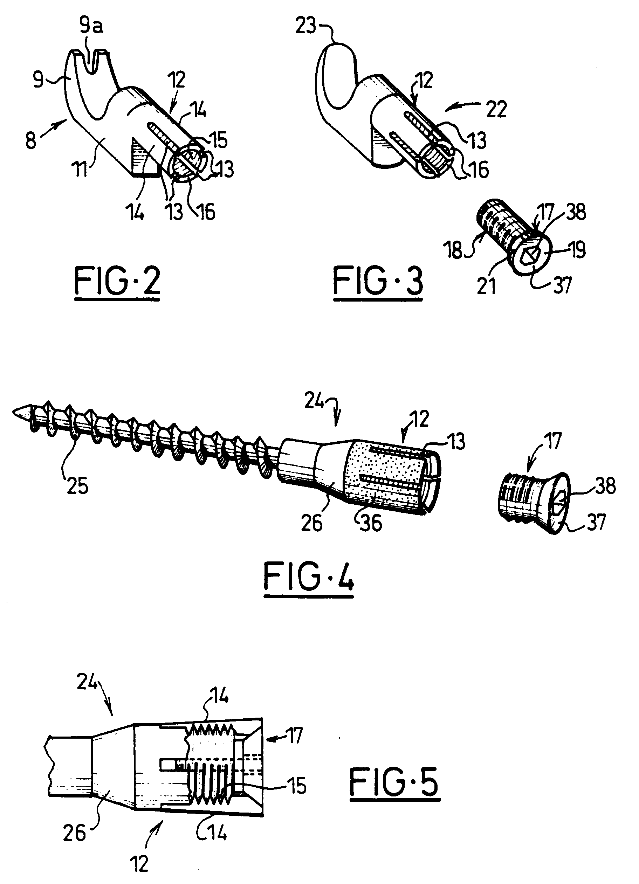

### Description des éléments constitutifs du dispositif représenté à la Fig.1 (Fig.2 à 14).

Les éléments d'ancrage osseux 1 peuvent être, soit des crochets (Fig.2 et 3), soit des vis pédiculaires (Fig.4 et 5).

Le crochet pédiculaire 8 (Fig.2) comprend une partie d'ancrage vertébral constituée par le crochet 9 proprement dit, dont le bord présente une entaille 9a, et qui est reliée à une partie médiane 11 prolongée par un corps cylindrique 12. Dans ce dernier sont ménagées au moins deux fentes longitudinales, à savoir quatre fentes 13 dans l'exemple représenté, qui délimitent entre elles quatre branches 14 expansibles radialement. Les faces intérieures de ces dernières présentent un taraudage 15, et un chanfrein annulaire 16 est formé sur les extrémités des branches 14.

Enfin le crochet 8 est complété par une vis d'expansion 17 comportant une tige filetée 18 adaptée pour pouvoir se visser dans le corps 12, et une tête 19 pourvue d'une surface conique 21. Cette dernière peut venir en appui sur le chanfrein 16 lorsque la vis 17 est vissée dans les taraudages 15, en provoquant une expansion radiale des branches 14, dont la surface externe affecte alors une forme tronconique.

La tête conique 19 de la vis 17 présente une surface plane 37, bordée par la zone conique 21, et dans laquelle est formée une empreinte centrale 38 adaptée pour recevoir une clé de serrage non représentée. L'empreinte 38 peut être de forme à la fois hexagonale et hexalobée.

Le crochet laminaire 22 de la Fig.3 diffère du crochet pédiculaire 8 uniquement par sa pointe d'ancrage 23 dont le bord est continu.

La vis pédiculaire 24 des Fig.4 et 5 comporte une tige filetée 25 d'ancrage osseux, prolongée du côté opposé à sa pointe par une partie conique 26 elle-même suivie par un corps cylindrique 12 similaire au corps 12 des Fig.2 et 3. A la fin du vissage de la vis 17 associée sur les taraudages 13, sa surface conique 21 vient s'appuyer sur le chanfrein 16 en provoquant une expansion radiale des branches 14 (Fig.5).

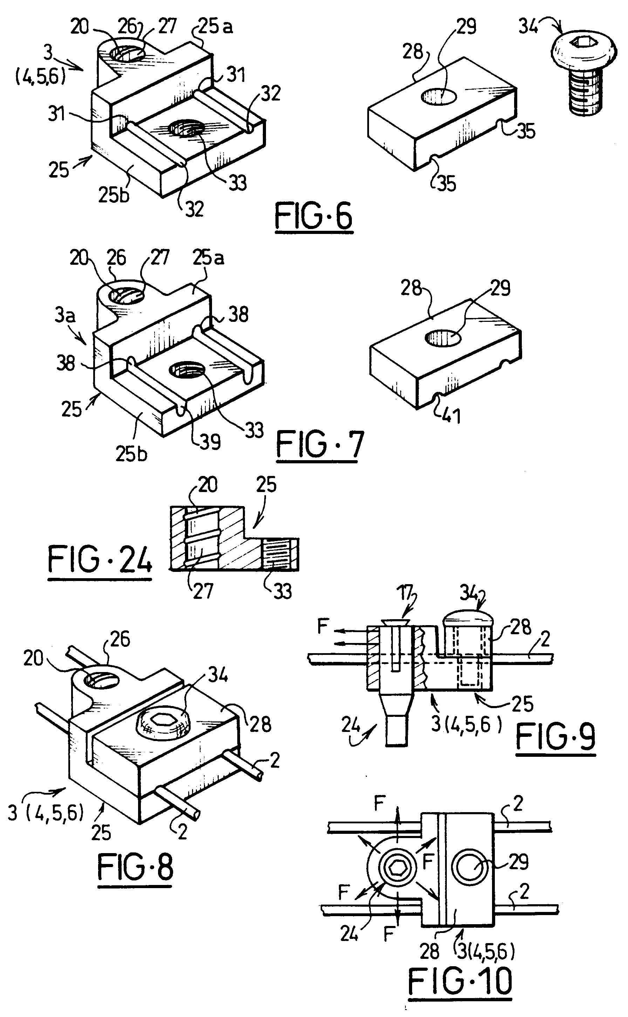

Chacun des connecteurs 3, 4, 5, 6 représentés à la Fig.1 peut être réalisé conformément à l'un des deux modes de réalisation représentés aux Fig.6 à 10 et 24.

Le connecteur 3 des Fig.6, 8, 9 et 10 comprend un socle 25 à profil en L, la petite branche 25a du L étant prolongée par une excroissance 26 dans laquelle est percé un trou taraudé 27. Dans la branche 25a sont usinés deux alésages 31, parallèles l'un à l'autre et qui traversent la branche 25a de part et d'autre de l'excroissance 26. Ces alésages 31 sont circulaires et s'étendent en section de part et d'autre du plan de la grande branche 25b du socle 25. Ils se prolongent par deux gouttières parallèles 32, semi-circulaires, ménagées dans la surface de la branche 25b du L, et qui s'étendent jusqu'au bord de cette dernière, dans la partie centrale de laquelle est percé un trou taraudé 33. Le connecteur 3 (4, 5, 6) comporte également une bride 28 constituée par une plaquette dans laquelle est percé un trou central 29, et qui peut venir se placer en appui sur les deux branches du L du socle 25.

Le connecteur 3 est complété par une vis 34 dont la tige filetée traverse les trous 29 et 33 lorsque la bride 28 est placée sur le socle 25. Sur l'une des faces de la bride 28 sont usinées deux gouttières 35 semi-circulaires et parallèles, et qui sont positionnées de telle sorte que lorsque la bride 28 est placée en appui sur la grande branche 25b du socle 25, ces gouttières 35 soient complémentaires des gouttières semi-circulaires 32 pour former des alésages circulaires, dans lesquels peuvent prendre place les tiges 2.

Le trou 27 est dimensionné pour pouvoir recevoir le corps 12 de l'élément d'ancrage 8, 22 ou 24, une vis 24 étant représentée engagée dans le trou 27 aux Fig.9 et 10. Lorsque la vis 17 est vissée dans le corps 12, celui-ci subit une expansion radiale (Fig.5) et ses branches 14 sont fortement appliquées sur la paroi du trou 27, en solidarisant par conséquent le connecteur 3 et l'élément d'ancrage 8, 22 ou 24. L'expansion radiale des branches 14 est provoquée par la force exercée par la zone conique 21 de la vis 17 sur le chanfrein 16 de même inclinaison. La surface extérieure du corps 12 présente avantageusement des aspérités 36 (Fig.4) la rendant rugueuse, ce qui favorise la solidarisation du corps 12 avec le connecteur. Une rainure hélicoïdale 20 est également ménagée dans le trou 27 du connecteur pour améliorer la liaison mécanique élément d'ancrage osseux/connecteur. Les forces radiales appliquées sur les parois de l'excroissance 26 par le serrage de la vis 17 sont symbolisées par les flèches F sur les Fig.9 et 10.

Les tiges flexibles élastiques 2, une fois convenablement positionnées dans les alésages 31 et les gouttières 32, 35, peuvent être fixées au connecteur 3 par vissage de la vis 34 dans les trous 29 et 33 (Fig.8 et 9). Avant serrage définitif par la vis 34, les éléments longitudinaux 2 peuvent coulisser ou translater et tourner autour de leur axe, ce qui présente un intérêt lors de leur mise en place, car cela permet de régler l'intervalle entre deux ancrages osseux, de vriller, de courber l'ensemble tiges flexibles + connecteurs dans toutes les positions pour s'adapter aux éléments d'ancrage et faciliter leur introduction dans le trou 27 du connecteur. Une fois cet intervalle ajusté, la liaison complète entre le connecteur 3 et les tiges longitudinales 2 est assurée par serrage de la bride 28 grâce à la vis 34.

Dans la variante d'exécution de la Fig.7, le connecteur 3a diffère du connecteur 3 uniquement par le fait que les alésages 38 et les gouttières 39, 41 respectivement formées sur le socle 25 et sur la bride 28, ont une section oblongue. Le grand axe de ces sections oblongues s'étend perpendiculairement à la face de la branche 25b du socle 25 et à la face correspondante de la bride 28. La forme oblongue des alésages 38 et des gouttières 39, 41 présente l'avantage d'augmenter l'inertie des tiges oblongues correspondantes (non représentées), et donc la résistance à la flexion dans le plan passant par l'axe de l'ancrage et parallèle aux tiges longitudinales.

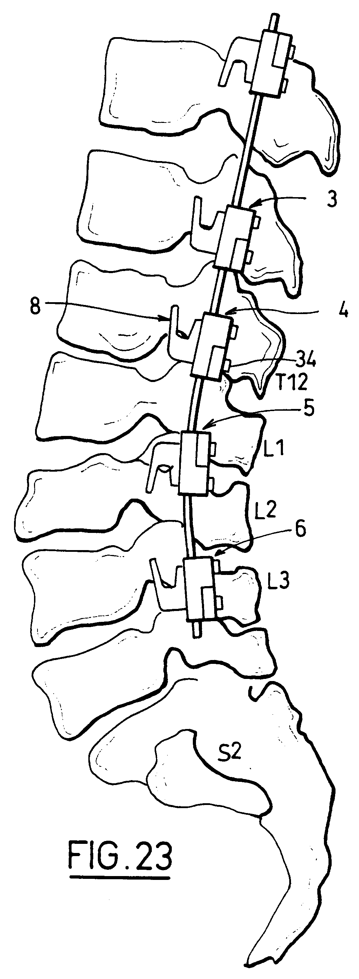

Les éléments de l'instrumentation décrite ci-dessus, une fois assemblés et mis en place sur un segment rachidien, se présentent comme illustré à la Fig.23 à titre d'exemple.

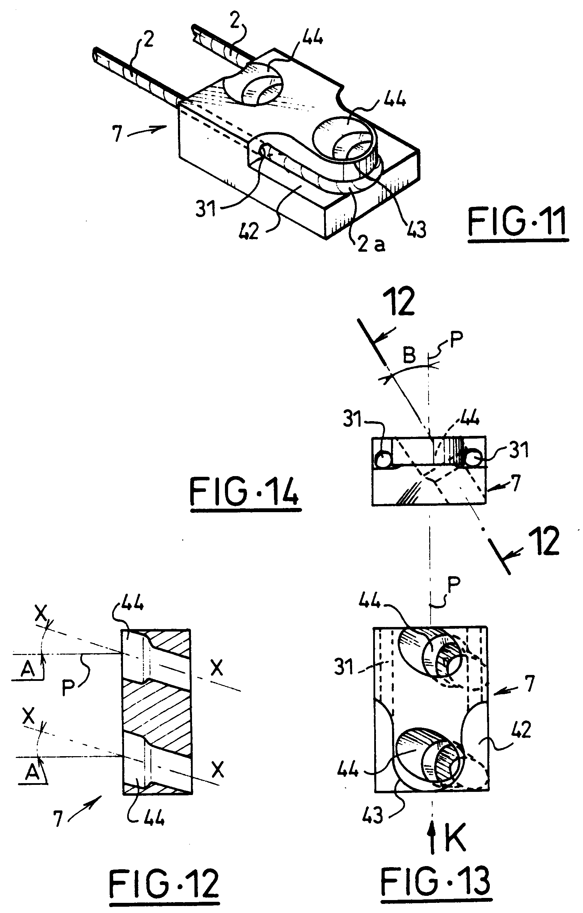

Le connecteur 7 de fixation sacrée (Fig.11) comporte, à une de ses extrémités, un dégagement 42 délimitant un épaulement arrondi 43, de préférence semi-circulaire, prolongé par deux alésages parallèles 31 qui percent de part en part le connecteur 7. Les deux tiges longitudinales 2 traversant les alésages 31 sont reliées par une boucle 2a en appui sur l'épaulement 43, et avec laquelle elles forment donc une tige longitudinale unique.

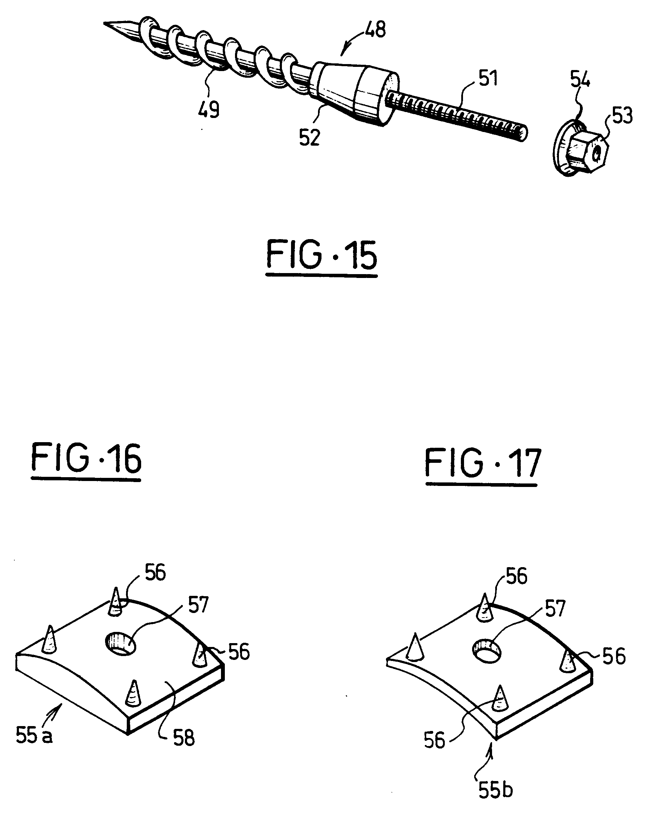

Dans le corps du connecteur 7, sont percés deux alésages 44, pour recevoir des vis non représentées (telles que 24) de fixation sacrée. Les axes XX des alésages 44 sont parallèles et leurs projections dans un plan axial longitudinal (P) du connecteur 7 ont une inclinaison (A) sur des perpendiculaires aux faces du connecteur contenues dans ledit plan (Fig.12 et 14). De plus les axes XX ont une inclinaison (B) sur le plan axial (P) (Fig.12).

L'angle A peut être par exemple de l'ordre de 15°, tandis que B peut être d'environ 30°, ces valeurs n'étant bien entendu pas limitatives. L'angle B peut être orienté à gauche ou à droite, afin de différencier une prise sacrée gauche d'une prise sacrée droite. Les alésages 44 permettent le bouclage sur l'arrondi 43 des éléments de liaison flexibles 2.

La mise en oeuvre et les avantages du montage lombo-sacré illustré à la Fig.1 sont les suivants.

Ce montage permet de stabiliser et d'arthrodéser la zone du rachis constituée des lombaires et du sacrum, essentiellement S1 et S2. Sa mise en place est réalisée à l'aide de vis pédiculaires 24 pour la zone lombaire et la vertèbre S1, et de vis sacrées 24 pour la vertèbre S2. Ces vis constituent les ancrages osseux, les vis sacrées étant des vis à os ayant un diamètre par exemple de 3,5mm, utilisées couramment en chirurgie orthopédique.

Les tiges longitudinales 2 sont en fait constituées, comme déjà indiqué, par une seule tige bouclée sur le connecteur sacré 7, les deux branches de la boucle 2a, constituées par les tiges 2, étant ensuite enfilées dans les connecteurs 3, 4, 5, 6. L'ensemble de l'instrumentation est rendu solidaire du rachis par les vis pédiculaires et sacrées en S2. L'ensemble du dispositif peut être cintré pour faire coïncider sa courbure avec la courbure du rachis. Le dispositif est bilatéral, seul le côté droit étant représenté à la Fig.1.

La technique chirurgicale de mise en place du dispositif est la suivante.

Les implants vissés ou ancrages sont mis en place dans un premier temps par les pédicules et dans S1. L'implantation des vis se fait "droit devant" ou plus latéralement sur la racine de la transverse, de façon à faire converger les deux vis pédiculaires d'une même vertèbre.

Le connecteur de fixation sacrée 7 et les connecteurs 3, 4... pour les vis pédiculaires et de S1 sont mis en place sur les éléments de liaison flexibles 2. Les brides 28 ne sont pas serrées, afin de permettre leur réglage en fonction des intervalles de vertèbre à vertèbre, ou de vis pédiculaire à vis pédiculaire et laisser une grande flexibilité. L'ensemble des éléments longitudinaux flexibles 2 et des connecteurs 3... est alors cintré pour s'adapter à la courbure du rachis (lordose ou cyphose). L'introduction est ensuite réalisée en enfilant les connecteurs sur les têtes de vis 24.

Les vis d'expansion 17 sont serrées à mesure de l'introduction de l'ensemble tige flexible 2- connecteurs 3, 4.., l'opération étant exécutée à partir de l'élément fonctionnel situé le plus haut sur le rachis vers le sacrum. Une fois l'ensemble des connecteurs introduit et verrouillé dans les têtes de vis, les vis sacrées de S2 sont mises en place dans le connecteur sacré spécifique 7.

Le côté opposé est instrumenté de façon identique. Les manoeuvres de réduction ou de correction sont alors réalisées étage par étage, avec verrouillage des brides respectives. Cette manoeuvre est faite en partant du sacrum de façon unilatérale ou bilatérale par rapprochement ou par écartement des connecteurs avec un matériel ancillaire approprié. Les greffons sont alors placés entre les différents espaces laissés par l'instrumentation afin de réaliser l'arthrodèse.

Grâce au fait que les têtes 12 des vis d'ancrage 24 sont dépourvues de butée axiale, il est possible de procéder, en variante, comme indiqué ci-dessous.

Dans la mesure où l'ensemble connecteurs plus éléments 2 de liaison flexible forme un tout, celui-ci peut être posé sur la face postérieure du rachis à la manière d'une plaque d'ostéosynthèse rachidienne. Les vis 24 sont alors introduites au travers des connecteurs laissés libres sur les éléments 2 de liaison flexible, afin d'effectuer l'ancrage pédiculaire. Les vis d'expansion 17 sont alors mises en place et vissées pour rendre les vis 24 solidaires des connecteurs 3. Le reste du montage s'exécute comme indiqué précédemment.

La vis 48 représentée à la Fig.15 est particulièrement adaptée à certaines pathologies du rachis, telles que spondylolistésis, spondylolyse.

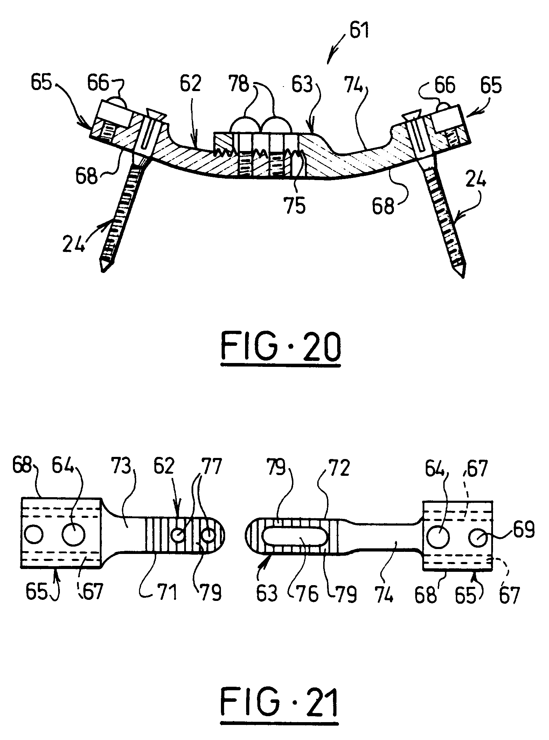

Elle est à double filetage et comporte donc une première tige filetée 49 d'ancrage osseux, du même type que la tige 25 des vis pédiculaires 24, et une seconde tige filetée 51 dans le même axe que la tige 49, dont elle est séparée par une tête 52. La tige 51 est adaptée pour recevoir un écrou 53 éventuellement pourvu d'une surface d'appui élargie 54. La vis 48 est utilisée avec un connecteur tel que 3, 4... ou une plaque de connecteur double 61 (Fig.20-21) qui sera décrit plus loin. Après ancrage osseux, le vissage de l'écrou 53 en appui sur le connecteur 3... ou la plaque du connecteur double 61, provoque un déplacement de la vertèbre d'avant en arrière suivant le plan sagittal. Enfin le chirurgien coupe la partie de la tige filetée 51 qui dépasse.

### Description d'une instrumentation antérieure (Fig.16 à 19)

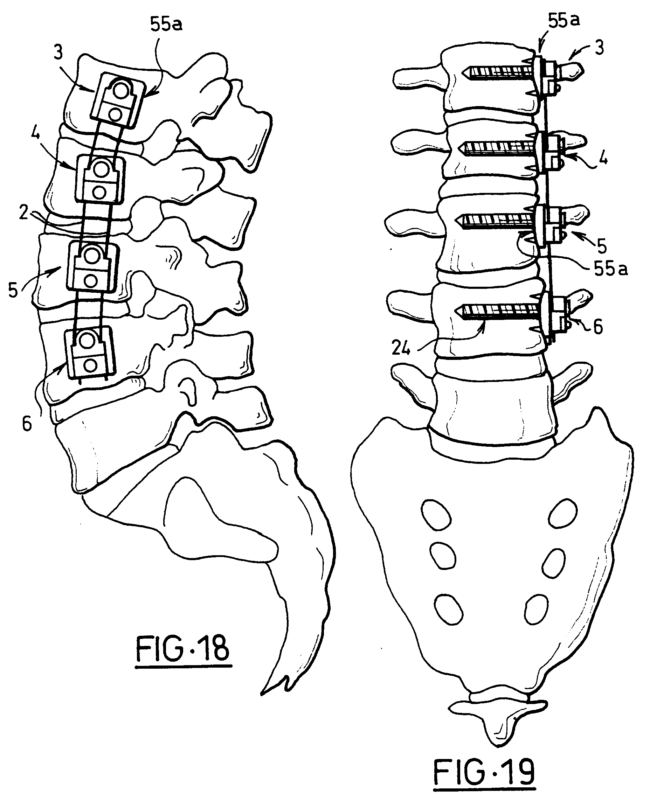

Certaines pathologies du rachis justifient l'utilisation d'instrumentations d'abord antérieur, dites instrumentations antérieures. Le dispositif selon l'invention est compatible avec ce type d'abord et de mise en place. Toutefois, il est alors avantageusement complété par des plaques d'appui antérieur 55a ou 55b (Fig.16 et 17) permettant d'éviter l'enfoncement des vis corporéales 24 correspondantes (Fig.19) et d'obtenir un meilleur alignement des connecteurs 3, 4, 5, 6 (le dispositif ne comportant pas dans cette utilisation de connecteur 7 de fixation sacrée).

En effet, le corps vertébral présente sur sa périphérie une concavité qui rend parfois délicate la mise en place de matériel. Une faible résistance de la corticale périphérique justifie également d'élargir l'appui sur le corps et d'éviter le contact direct du cône 26 de la vis 24.

Afin de faciliter sa mise en place, chaque plaque d'appui 55 est pourvue de picots coniques 56, striés ou non, assurant son positionnement et sa stabilité tant que la vis corporéale 24 n'est pas mise en place. Chaque plaque 55 est percée d'un trou central 57 de passage de la vis corporéale 24. La plaque 55 est soit rigide (plaque 55a de la Fig.15) et pour cela d'épaisseur suffisante, avec un galbe 58 adapté à la concavité de la vertèbre, soit souple (plaque 55b de la Fig.16), et dans ce cas de plus faible épaisseur que la plaque 55a, afin de pouvoir s'adapter à la concavité de la vertèbre.

Les autres éléments de l'instrumentation sont identiques en tous points à ceux de l'instrumentation postérieure décrite ci-dessus, excepté le fait qu'il n'y a pas de plaque ou prise sacrée. L'ensemble du dispositif mis en place sur le rachis est représenté aux Fig.18 et 19.

### Description du dispositif adapté à l'instrumentation des fractures (Fig.20 à 22)

L'instrumentation de fractures corporéales rend parfois impossible le vissage pédiculaire de la ou des vertèbres fracturées. Il est donc nécessaire de passer un étage ou élément fonctionnel vertébral. Cette fonction est remplie par le connecteur double 61 (Fig.20 et 21) adapté pour former un pont au-dessus d'une vertèbre fracturée.

Le connecteur double 61 comprend deux plaques 62 et 63. Ces deux plaques présentent un cintrage approprié afin de respecter la courbure anatomique du patient, et comportent chacune une bride terminale 65 munie d'un élément (vis) de serrage 66 permettant, de manière similaire à ce qui a été décrit précédemment, de bloquer les tiges flexibles 2 dans leurs alésages 67 ménagés dans le socle 68 d'appui des brides respectives 65. A cet effet ces socles 68 sont percés chacun d'un trou 69 de passage de la tige filetée de la vis de blocage 66, en plus des trous 64 de passage des tiges des vis d'ancrage osseux 24.

Chaque plaque 62, 63 comprend de plus une partie terminale allongée 71, 72 respective, reliée au socle terminal 68 par un raccord 73, 74. Ce dernier présente un décrochement 75 permettant à l'extrémité 72 de venir s'appliquer sur la surface de l'extrémité 71 de la plaque 62. Dans l'extrémité 72 est agencé un trou oblong 76, et dans l'extrémité allongée 71 est ménagé au moins un trou 77 (deux trous 77 dans l'exemple représenté) de telle sorte que le trou oblong 76 puisse venir se placer en vis-à-vis des trous 77. L'assemblage des deux plaques 62 et 63 peut ainsi être réalisé au moyen de deux vis 78 traversant le trou oblong 76 et les trous 77 pour bloquer les deux plaques l'une sur l'autre (Fig.20).

Des aspérités sont formées sur les surfaces des plaques 62, 63 en appui mutuel, c'est-à-dire les surfaces des extrémités allongées 71 et 72. Dans l'exemple décrit, ces aspérités sont constituées par des crantages 79 formés autour du trou oblong 76 et des trous 77.

Le trou oblong 76 permet de régler la position relative des deux plaques 62, 63, et les aspérités 79 assurent la stabilité relative des deux plaques en translation et en rotation.

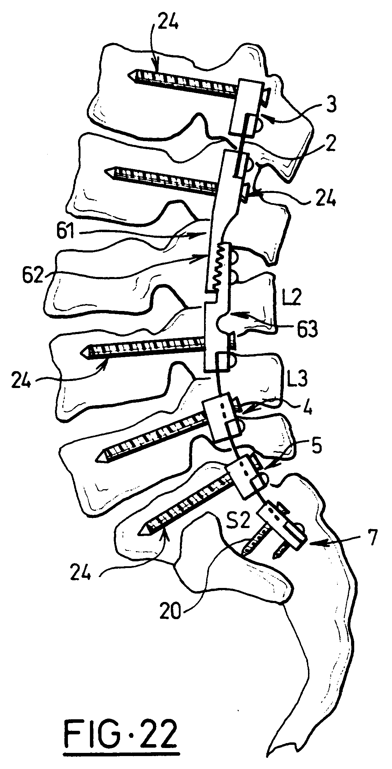

Le mode opératoire de mise en place de l'instrumentation comportant des connecteurs doubles 61 (Fig.22) est le suivant.
a) mise en place des vis pédiculaires 24 et de S1.
b) mise en place de l'ensemble constitué par les connecteurs simples 3, 4, 5, le connecteur sacré 7 et un connecteur double 61, disposé entre les connecteurs 3 et 4, (le connecteur 61 est dimensionné pour enjamber une vertèbre fracturée L2), par enfilement sur les corps 12 des vis pédiculaires 24, puis on serre ces dernières dans les connecteurs 3, 61, 4, 5.
c) réduction de la fracture par manoeuvre des corps de vis 24 de part et d'autre du foyer de fracture.
d) fixation et serrage des deux vis 78 de réglage du connecteur double 61.
e) serrage des brides 65 de fixation des tiges 2.
f) mise en place des vis 20 en S2.

Les deux dernières opérations peuvent être permutées.

On obtient alors un montage mixte de rigidité accrue dans le foyer de fracture, par l'intermédiaire du connecteur double 61, et plus élastique de part et d'autre du foyer, ce qui assure une stabilité globale du rachis.

Bien entendu on utilise les crochets 8 et 22 lorsque cela est approprié, les montages illustrés aux dessins n'étant fournis qu'à titre d'exemples.

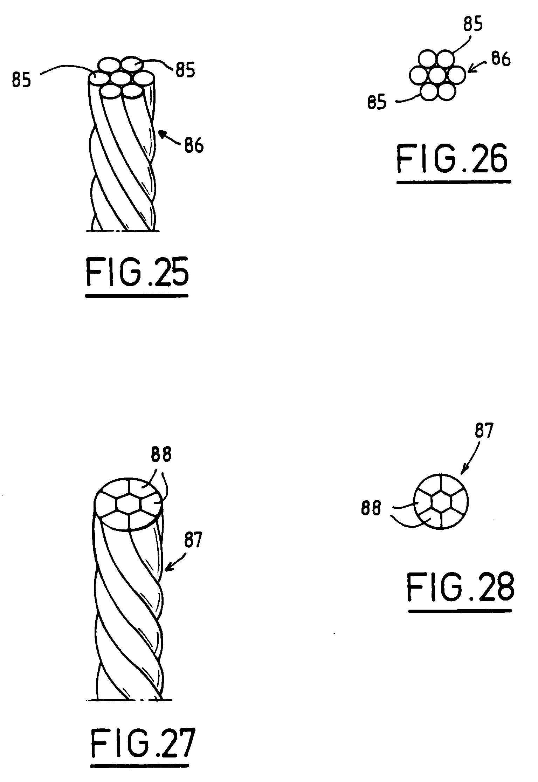

Les Fig. 25 et 26 illustrent une autre forme de réalisation des tiges flexibles longitudinales, ici constituées chacune, non par une tige pleine telle que 2, mais par un assemblage de fils 85 formant un toron 86. Dans cet exemple le toron 86 est ainsi réalisé par assemblage de 7 fils 85, et peut être rétreint.

Les tiges ou torons 86 doivent reprendre les efforts en traction et compression et permettre une certaine flexibilité pour reprendre les déplacements imposés. L'utilisation d'un toron ou d'une tige pleine peut améliorer la tenue en fatigue par flexion de l'élément longitudinal. En effet, la tige 86 peut avoir un diamètre par exemple de 2mm ou 2,5mm. Il est évident qu'un toron, par exemple de 7 fils, aura des diamètres de fils réduits, qui lui confèreront une grande flexibilité.

Dans la variante de réalisation des Fig.27-28, le toron 87 est constitué par l'assemblage de 7 fils 88 (un fil central et six fils périphériques) retréfilés pour accroître la section métallique et la rigidité du toron.

Les torons 86, 87 peuvent comporter un nombre variable de fils, et être réalisés par exemple en acier inoxydable 316L à haute limite élastique.

L'utilisation de tiges pleines 2 ou de torons 86, 87 est identique. L'amélioration de la flexibilité apportée par les torons se fait au détriment de la tenue en compression. Par conséquent le choix d'une tige pleine ou d'un toron, et sa composition, est un compromis en fonction des indications.

Ce dispositif peut être utilisé en abord postérieur ou antérieur. L'ensemble des tiges et des connecteurs, grâce à la flexibilité des tiges, permet de s'adapter aisément et sans contrainte, à tout positionnement des éléments d'ancrage osseux. Le glissement des connecteurs sur les tiges après fixation des éléments d'ancrage osseux à ceux-ci, permet d'affiner la réduction du segment rachidien instrumenté. La fixation par bridage des connecteurs sur les tiges, rigidifie l'ensemble tout en préservant une certaine élasticité à l'instrumentation de par les caractéristiques mécaniques des tiges, favorisant ainsi la prise de la greffe osseuse associée à l'ostéosynthèse.

Les vis vertébrales peuvent être introduites avant les éléments de liaison ou à travers.

## Revendications

1. Dispositif d'ostéosynthèse rachidienne, comportant :
- des éléments (8; 22; 24) d'ancrage osseux par exemple vis vertébrales, crochets,... chaque élément comprenant une partie d'ancrage vertébral (9, 23, 25), un corps cylindrique (12) dans lequel sont ménagées au moins deux fentes longitudinales (13) délimitant entre elles au moins deux branches (14) ainsi qu'un taraudage (15), et une vis d'expansion interne (17), adaptée pour pouvoir être vissée à l'intérieur du corps en provoquant un écartement radial des branches,
caractérisé en ce qu'il comprend :
- des tiges longitudinales (2), flexibles en condition d'utilisation sur un patient,
- et des connecteurs (2, 4, 5, 6, 7, 61) adaptés pour pouvoir être solidarisés avec des éléments d'ancrage correspondants et les tiges, chaque connecteur comprenant un socle à profil en L (25) percé de part en part de deux alésages (31) et d'un trou (27) de réception du corps expansible (12) d'un élément d'ancrage (8, 22, 24), des gouttières (32) étant ménagées sur ledit socle dans le prolongement desdits alésages, le connecteur étant pourvu d'une bride (28) d'immobilisation des tiges pouvant être fixée sur le socle par u n organe (34) de serrage et sur une face de laquelle sont formées des gouttières (35) complémentaires des gouttières du socle, et les tiges étant logées dans les alésages et les gouttières; l'ensemble du dispositif constituant une structure flexible en condition d'utilisation sur un patient.

2. Dispositif selon la revendication 1, caractérisé en ce que les tiges sont à haute limite élastique et à haute résistance à la rupture.

3. Dispositif selon la revendication 2, caractérisé en ce que les alésages (31) et les gouttières (32), sont soit circulaires, soit non circulaires par exemple oblongs.

4. Dispositif selon l'une des revendications 1 à 3, caractérisé en ce qu'il comprend un connecteur (7) de fixation sacrée pourvu de deux alésages (44) d'axes (XX) parallèles, dont les projections dans un plan axial longitudinal (P) du connecteur ont une inclinaison (A) sur des perpendiculaires aux faces du connecteur (7) contenues dans ledit plan (P), et les axes (XX) ont une inclinaison (B) sur ledit plan axial (P).

5. Dispositif selon la revendication 4, caractérisé en ce que le connecteur sacré (7) est pourvu d'un épaulement arrondi (43) dont les extrémités débouchent dans des alésages (31) de passage d'une boucle (2a) de liaison entre les deux tiges (2).

6. Dispositif selon l'une des revendications 1 à 3, caractérisé en ce qu'il comporte des plaques (55a, 55b) d'appui antérieur sur le rachis, pourvues chacune de moyens (56) de fixation sur la vertèbre correspondante, adaptées pour pouvoir épouser la concavité de ladite vertèbre, et percées chacune d'un trou (57) de passage de l'élément d'ancrage osseux (8, 22, 24) traversant le connecteur (3..) monté sur ladite plaque d'appui.

7. Dispositif selon la revendication 6, caractérisé en ce que chaque plaque est soit rigide (55a) avec un galbe (58) adapté à la concavité de la vertèbre associée, soit (55b) d'épaisseur suffisamment faible pour la rendre souple afin de lui permettre de s'adapter à ladite concavité.

8. Dispositif selon l'une des revendications 1 à 7, caractérisé en ce qu'il comprend au moins un connecteur (61) adapté pour former un pont au-dessus d'une vertèbre fracturée, comportant deux plaques (62, 63) percées chacune d'un alésage de passage d'un élément d'ancrage osseux (24), pourvues de brides (65) de fixation des tiges (2), de moyens de réglage longitudinal des plaques l'une par rapport à l'autre et (78) d'assemblage desdites plaques entre elles, l'une (63) des plaques présentant un décrochement (75) pour permettre à sa partie terminale (72) de venir en appui sur la partie terminale correspondante (71) de l'autre plaque (62).

9. Dispositif selon la revendication 8, caractérisé en ce que lesdits moyens de réglage et de fixation comprennent un trou oblong (76) agencé dans l'une (63) des plaques, au moins un trou taraudé (77) dans l'autre plaque (62) en regard du trou oblong, et une vis (78) d'assemblage, les plaques étant cintrées pour s'adapter à la courbure anatomique du patient en étant assemblées dans le prolongement l'une de l'autre.

10. Dispositif selon la revendication 9, caractérisé en ce que des aspérités (79) sont formées sur les surfaces des plaques (62, 63) en appui l'une sur l'autre, par exemple des crantages de part et d'autre du trou oblong (76) et du trou (77) de passage de la vis.

11. Dispositif selon l'une des revendications 1 à 10, caractérisé en ce que les tiges flexibles (2) sont de section circulaire ou autre,par exemple oblongue.

12. Dispositif selon l'une des revendications 1 à 11, caractérisé en ce que les tiges flexibles sont en matériau biocompatible.

13. Dispositif selon la revendication 1, caractérisé en ce que le trou (27) de réception du corps expansible dans le connecteur comporte une rainure hélicoïdale (20) améliorant la fixation mécanique des éléments d'ancrage aux connecteurs.

14. Dispositif selon l'une des revendications 1 à 13, caractérisé en ce que les vis vertébrales (24) sont dépourvues de butée axiale.

15. Dispositif selon l'une des revendications 1 à 14, caractérisé en ce que les tiges longitudinales flexibles sont constituées par des assemblages de fils (85; 88) formant par exemple des torons (86; 87).

16. Dispositif selon la revendication 15, caractérisé en ce que le toron (86; 87) est formé de fils rétreints (85) ou retréfilés (88).

## Claims

1. A spinal osteosynthesis device comprising:
- bone anchorage elements (8; 22; 24), for example, vertebral screws, hooks,.. , each element comprising a vertebral anchorage portion (9, 23, 25), a cylindrical body (12) wherein there are arranged at least two longitudinal slots (13) delimiting between them at least two arms (14), as well as an internal thread (15), and an internal expansion screw (17), made suitable for being screwed inside the body while producing a radial divergence of the arms,
characterized in that it comprises:
- longitudinal rods (2) flexible in their state of use in a patient,
- and connectors (2, 4, 5, 6, 7, 61) adapted to allow them to be joined to corresponding anchorage elements and the rods, each connector comprising an L-shaped base component (25), pierced on either side by two bores (31) and a hole (27) for receiving the expandable body (12) of an anchorage element (8; 22; 24), grooves (32) being arranged in the said base component in the extension of the said bores, the connector being provided with a clamp part (28) for fixing the rods, which can be secured on the base component by a tightening element (34), and on one side thereof there are formed grooves complementary to the grooves of the base component, and the rods being accommodated in the bores and the grooves; the whole of the device forming a flexible structure in its state of use in a patient.

2. A device according to claim 1, characterized in that the rods have a high elastic yield point and a high breaking strength.

3. A device according to claim 2, characterized in that the bores (31) and the grooves (32) are either circular or non-circular, for example, oblong.

4. A device according to one of claims 1 to 3, characterized in that it comprises a sacral fixing connector (7) provided with two bores (44) with parallel axes (XX) whose projections in a longitudinal axial plane (P) of the connector has an inclination (A) to the perpendiculars to the sides of the connector (7) contained in the said plane (P), and the axes (XX) have an inclination (B) to the said axial plane (P).

5. A device according to claim 4, characterized in that the sacral connector (7) is provided with a rounded shoulder (43) whose ends open out in bores (31) for the passing of a loop (2a) for the connection between the two rods (2).

6. A device according to one of claims 1 to 3, characterized in that it comprises anterior bearing plates (55a, 55b) on the spine, each provided with fixing means (56) on the corresponding vertebra, adapted to be capable of assuming the shape of the concavity of the said vertebra, and each pierced by a hole (57) for the passing of the bone anchorage element (8, 22, 24) passing through the connector (3..) mounted on the said bearing plate.

7. A device according to claim 6. characterized in that each plate is either rigid (55a) with a contour (58) adapted to the concavity of the associated vertebra, or (55b) of a sufficiently small thickness to render it flexible so as to allow it to fit the said concavity.

8. A device according to one of claims 1 to 7, characterized in that it comprises at least one connector (61) adapted to form a bridge above a fractured vertebra, comprising two plates (62, 63) each pierced by a bore for the passing of a bone anchorage element (24), provided with securing parts (65) for fixing the rods (2), means for the longitudinal adjustment of the plates with respect to one another, and means (78) for the connection of the said plates to one another, one (63) of the plates having a set back (75) to allow its end portion (72) to come to bear on the corresponding end portion (71) of the other plate (62).

9. A device according to claim 8, characterized in that the said adjustment and fixing means include a slot (76) arranged in one (63) of the plates, at least one threaded hole (77) in the other plate (62) opposite the slot, and a connecting screw (78), the plates being curved to adapt to the patient's anatomical curve by being joined in one another's extension.

10. A device according to claim 9, characterized in that asperities (79) are formed on the surfaces of the plates (62, 63) bearing on one another, for example, serrations on either side of the slot (76) and of the hole (77) for the passing of the screw.

11. A device according to one of claims 1 to 10, characterized in that the flexible rods (2) have a circular or other cross section, for example, an oblong cross section.

12. A device according to one of claims 1 to 11, characterized in that the flexible rods are made of a bio-compatible material.

13. A device according to claim 1, characterized in that the hole (27) for receiving the expandable body in the connector has a helical groove (20) improving the mechanical fixing of the anchorage elements to the connectors.

14. A device according to one of claims 1 to 13, characterized in that the vertebral screws (24) are without an axial stop.

15. A device according to one of claims 1 to 14, characterized in that the flexible longitudinal rods are constituted by the assembly of wires (85; 88) forming for example strands (86; 87).

16. A device according to claim 15, characterized in that the strand (86; 87) is formed of swaged wires (85) or redrawn wires (88).

## Patentansprüche

1. Vorrichtung zur Osteosynthese von Rückenwirbeln, bestehend aus:
- Elementen (8;22;24) zur Verankerung am Knochen, z.B. Wirbel-Schrauben, Haken, ..., wobei jedes Element ein Teil zur Verankerung an einem Wirbel (9,23,25), ein zylindrisches Teil (12), in dem sich mindestens zwei Längsschlitze (13), die zwischen sich mindestens zwei Arme (14) begrenzen, sowie ein Gewinde (15) befinden, und eine Schraube (17) zur Expansion von innen aufweist, die in das Innere des Körpers geschraubt werden kann und dabei ein radiales Auseinanderspreizen der Arme verursacht,
gekennzeichnet durch
- Stangen (2), die bei Anwendung bei einem Patienten biegsam sind, und
- Verbindungselemente (2,4,5,6,7,61), die mit entsprechenden Verankerungselementen und den Stangen verbunden werden können, wobei jedes Verbindungselement einen im Profil L-förmigen Sockel (25) aufweist, der von zwei Bohrungen (31) vollständig und von einem Loch (27) zur Aufnahme eines dehnbaren Körpers (12) eines Verankerungselementes (8,22,24) durchbohrt ist, Schienen (32) in dem Sockel in Verlängerung der Bohrungen vorgesehen sind, das Verbindungselement mit einem Spannelement (28) ausgerüstet ist, die Stangen auf dem Sockel mittels eines Spannorgans (34) befestigt werden können, auf einer Fläche eines Spannelements Schienen (35) angeordnet sind, die mit den Schienen des Sockels zusammenwirken, und die Stangen in den Bohrungen und den Schienen sitzen, und wobei die gesamte Vorrichtung eine flexible Struktur bei Anwendung bei einem Patienten bildet.

2. Vorrichtung nach Anspruch 1, dadurch gekennzeichnet, daß die Stangen von hoher Elastizität sind und einen großen Bruchwiderstand haben.

3. Vorrichtung nach Anspruch 2, dadurch gekennzeichnet, daß die Bohrungen (31) und die Schienen (32) kreisförmig und nicht kreisförmig, z.B. länglich, sind.

4. Vorrichtung nach einem der Ansprüche 1 bis 3, dadurch gekennzeichnet, daß diese ein Verbindungselement (7) zur Sacrum-Befestigung aufweist, bei dem zwei Bohrungen (44) mit parallelen Achsen (XX) vorgesehen sind, deren Projektionen in einer längsaxialen Ebene (P) des Verbindungselementes eine Neigung (A) zu den Senkrechten der Flächen des Verbindungselementes (7) haben, die in der Ebene (P) liegen, wobei die Achsen (XX) eine Neigung (B) zur axialen Ebene (P) haben.

5. Vorrichtung nach Anspruch 4, dadurch gekennzeichnet, daß das Sacrum-Verbindungselement (7) mit einem abgerundeten Vorsprung (43) versehen ist, dessen Enden in die Bohrungen (31) über eine Verbindungsschlinge (2a) zwischen den beiden Stangen (2) übergehen.

6. Vorrichtung nach einem der Ansprüche 1 bis 3, dadurch gekennzeichnet, daß diese Platten (55a,55b) älterer Art zum Abstützen auf dem Rückenwirbel aufweist, daß jede mit Mitteln (56) zum Befestigen auf dem entsprechenden Wirbel versehen ist, daß sie zum Anpassen an die konkave Form des Wirbels geeignet sind, wobei jede Platte ein Loch (57) zum Durchtritt eines Knochen-Verankerungselementes (8,22,24) aufweist, das das Verbindungselement (3...) durchdringt, das auf der Abstützplatte befestigt ist.

7. Vorrichtung nach Anspruch 6, dadurch gekennzeichnet, daß jede Platte (55a) steif ist und eine Rundung aufweist, die der konkaven Form des zugeordneten Wirbels angepaßt ist, oder eine geeignete geringe Dicke hat (55b), so daß sie biegsam ist, um sie der konkaven Form des Wirbels anzupassen.

8. Vorrichtung nach einem der Ansprüche 1 bis 7, dadurch gekennzeichnet, daß diese mindestens ein Verbindungselement (61) aufweist, das eine Brücke über einen Wirbelbruch bildet, bestehend aus zwei Platten (62,63), von denen jede eine Bohrung für ein Knochen-Verankerungslement (24), ein Spannelement (65) zur Befestigung der Stangen(2), Mittel zur Regelung in Längsrichtung der Platten zueinander und Mittel (78) zur Befestigung der Platten untereinander aufweist, wobei eine Platte (63) Verhakungen (75) aufweist, die an einem Ende (72) eine Abstützung an dem entsprechenden Ende (71) der anderen Platte (62) ermöglicht.

9. Vorrichtung nach Anspruch 8, dadurch gekennzeichnet, daß die Mittel zur Regelung und Befestigung ein Langloch (76) in einer der Platten (63), mindestens eine Gewindebohrung (77) in der anderen Platte (62) gegenüber dem Langloch und eine Befestigungsschraube (78) aufweisen und daß die Platten gebogen sind, so daß sie im zusammengefügten Zustand in der Verlängerung der einen Platte in Richtung auf die andere der anatomischen Beugung des Patienten angepaßt sind.

10. Vorrichtung nach Anspruch 9, dadurch gekennzeichnet, daß auf den Oberflächen der Platten (62,63) Unebenheiten (79), die aneinander liegen, vorgesehen sind, z.B. Verzahnungen auf beiden Seiten des Langloches (76) und des Loches (77) für die Schraube.

11. Vorrichtung nach einem der Ansprüche 1 bis 10, dadurch gekennzeichnet, daß die biegsamen Stangen (2) einen kreisförmigen oder einen anderen, z.B. länglichen, Querschnitt haben.

12. Vorrichtung nach einem der Ansprüche 1 bis 11, dadurch gekennzeichnet, daß die biegsamen Stangen aus biokompatiblem Material bestehen.

13. Vorrichtung nach Anspruch 1, dadurch gekennzeichnet, daß das Loch (27) in dem Verbindungselement zur Aufnahme des expansiblen Körpers eine schraubenlinienförmige Nut (20) besitzt, die die mechanische Befestigung der Verankerungselemente am Verbindungselement verbessert.

14. Vorrichtung nach einem der Ansprüche 1 bis 13, dadurch gekennzeichnet, daß die Wirbelschraube (24) mit einem Anschlag in axialer Richtung versehen ist.

15. Vorrichtung nach einem der Ansprüche 1 bis 14, dadurch gekennzeichnet, daß die biegsamen Stangen aus zusammengefügten Fasern (85;88) bestehen, die z.B. Litzen (86;87) bilden.

16. Vorrichtung nach Anspruch 15, dadurch gekennzeichnet, daß die Litze (86;87) aus nicht-ausfüllenden Fasern (85) oder ausfüllenden Fasern (88) besteht.
